# EUROPEAN PATENT SPECIFICATION

(11) **EP 3 668 482 B1**
(45) Date of publication and mention of the grant of the patent: **17.01.2024**
(21) Application number: 18759683.8
(22) Date of filing: 15.08.2018
(51) Int. Cl.: A61K 8/49, A61K 8/63, A61Q 7/00, A61K 9/00, A61P 17/14, A61K 9/48, A61K 31/19, A61K 31/50, A61K 31/55, A61K 31/57, A61K 36/87, A61K 45/06

(54) **COMBINATION THERAPY FOR HAIR LOSS**
KOMBINATIONSTHERAPIE FÜR HAARAUSFALL
THÉRAPIE COMBINÉE POUR LA PERTE DE CHEVEUX

(30) Priority: 16.08.2017 GB 201713113
(43) Date of publication of application: 24.06.2020
(73) Proprietor: Eccles, Nyjon Karl, London W1G 8QW (GB)
(72) Inventor: Eccles, Nyjon Karl, London W1G 8QW (GB)
(74) Representative: Henderson, Helen Lee
(86) International application number: PCT/GB2018/052315
(87) International publication number: WO 2019/034871

(56) References cited:
- WO-A2-2013/078259
- US-A1- 2004 096 420
- US-A1- 2008 051 351
- US-A1- 2016 346 183
- ZENILDO SANTOS ET AL: "Drug discovery for alopecia: gone today, hair tomorrow", EXPERT OPINION ON DRUG DISCOVERY, vol. 10, no. 3, 9 February 2015 (2015-02-09), pages 269-292, XP055480330, London, GB ISSN: 1746-0441, DOI: 10.1517/17460441.2015.1009892
- MATIAS J R ET AL: "The effect of testosterone, cyproterone acetate, and minoxidil on hair loss in the androchronogenetic alopecia mouse", CLINICS IN DERMATOLOGY, J.B. LIPPINCOTT, PHILADELPHIA, PA, US, vol. 6, no. 4, 1 October 1988 (1988-10-01) , pages 169-176, XP026188309, ISSN: 0738-081X, DOI: 10.1016/0738-081X(88)90083-1 [retrieved on 1988-10-01]

## Description

### Field of Invention

The present invention relates to a combination therapy for use in treating hair loss. Cosmetic methods for reducing hair loss or stimulating hair growth or regrowth are also provided.

### Background to the Invention

Normally, all humans shed around 50 to 100 of the more than 100,000 hairs on the scalp each day. But problems begin when too many follicles enter rest phase permanently or are damaged or killed. When this happens, the rate of replacement may not keep up, eventually causing the hair to thin out visibly. This excessive hair loss is called alopecia.

Androgenetic alopecia (AGA), or male pattern hair loss is a genetically determined progressive condition with various ages of onset and rates of progression. Although prevalence increases with age, AGA is potentially reversible, particularly if treated early. In both men and women androgens, especially dihydrotestosterone (DHT), have been shown to play a critical role in the hair loss process. DHT is the most potent of the circulating androgens in human plasma and a major testosterone metabolite in human skin. The problem is that DHT extends the telogen (non-growing) phase of the hair cycle. As a result, the growth cycle (anagen) is shorter and it cannot keep up with the loss of hair.

The enzyme that converts testosterone to DHT is 5o-reductase and the important role of 5o-reductase activity in AGA is highlighted by the absence of hair loss in men with a congenital deficiency for a 5a-reductase enzyme. Furthermore, the success of 5o-reductase inhibitors, such as finasteride, in treating hair loss further supports the targeting of 5o-reductase in AGA.

In addition, the popular use of topical minoxidil highlights another possibility in therapy; to increase/improve the circulation to the scalp.

Clinical trials comparing the two approaches as monotherapies (minoxidil vs finasteride) have generally come to the conclusion that both are effective but that finasteride is the more efficacious treatment option. Since these single therapies have only achieved limited hair regrowth, combination therapies have been emerging as the treatment of choice for most dermatologists. Results have shown that finasteride in combination with either topical minoxidil or ketoconazole was significantly more efficacious than finasteride monotherapy with no differences in the incidence of adverse effects that were observed.

Disappointingly, however, finasteride has been associated with significant sexual side effects including decreased libido, erectile dysfunction, and ejaculation disorder. These symptoms of sexual dysfunction have been reported to persist even after stopping finasteride treatment. Incidences of male breast cancer and depression have also been reported.

While minoxidil represents a safer treatment for hair loss, as noted above, monotherapies have achieved somewhat limited results. Reportedly only 50% of users achieve hair growth after daily use for four months and hair regrowth tends to be vellus hair (non-adult, baby hairs). Santos et al. (Expert Opinion on Drug Discovery, 2015 vol. 10, no. 3, pages 269-292) reviews the effectiveness of minoxidil as a topical composition in the context of Rogaine^{®}.

Topical compositions comprising minoxidil, either a 5a-reductase inhibitor or an androgen receptor antagonist, and a prostaglandin analogue for topical application to the scalp have been described in US 2016/346183 but were not used in combination with an enteric composition. Similarly, topical compositions comprising, inter alia, minoxidil and progesterone have been described in US 2008/051351 but were not used in combination with an enteric composition. Oral supplements containing minoxidil in combination with other agents for increasing hair growth have been described in US 2004/096420 but were not used in combination with a topical composition.

Consequently, there is a need for alternative therapies that are effective in treating hair loss in both men and women.

### Summary of the Invention

Accordingly, in first aspect the present invention provides a combination therapy for use in treating hair loss comprising: (a) a topical composition comprising a bimatoprost and progesterone; and (b) an enteric composition comprising minoxidil, L-citrulline, grape seed extract and zinc.

The present invention additionally provides a cosmetic method for reducing hair loss or stimulating hair growth or regrowth. The method comprises administering a combination therapy comprising: (a) a topical composition comprising a bimatoprost and progesterone; and (b) an enteric composition comprising minoxidil, L-citrulline, grape seed extract and zinc to a subject in need thereof.

The invention is further defined in the appended set of claims.

Without being bound by theory, the inventor believes that the combination therapy of the present invention acts to reduce DHT levels in the scalp, improve circulation in the scalp and reduce inflammation and oxidative stress in the hair follicle, thereby providing an effective therapy for the treatment of hair loss. Current evidence suggests that noticeable hair regrowth of non-vellus hair can be seen in as little as two months. Additionally, the combination therapy of the present invention appears to reduce or avoid the side effects associated with finasteride treatment.

### Description

The present invention provides a combination therapy for use in treating hair loss. As used herein, hair loss may refer to alopecia including male-pattern hair loss, female-pattern hair loss, alopecia areata, traction alopecia and telogen effluvium. The combination therapy of the present invention can reduce or prevent hair loss and stimulate hair growth and/or regrowth. Treating hair loss as used herein therefore refers to both reducing the rate of loss of hair and stimulating hair growth and/or regrowth. Preferably hair regrowth as used herein refers to the growth of non-vellus hair.

The combination therapy comprises topical and enteric compositions. The topical composition comprises bimatoprost and progesterone.

The bimatoprost (a prostaglandin analogue) may be natural or synthetic. Prostaglandins have been used to reduce intra-ocular pressure in glaucoma and in patients using ophthalmic prostaglandins such as travoprost and latanoprost, it has been noted that there had been an increase in diameter, density and length of eyelashes. Bimatoprost has also been shown to significantly increase eyelash length. Prostaglandin analogues such as bimatoprost are believed to increase hair growth by increasing the percent of hairs in, and the duration of, the anagen or growth phase.

Topical compositions of invention typically comprise at least about 0.05 % (w/v) bimatoprost or at least about 0.1 % (w/v) bimatoprost or at least about 3 % (w/v) bimatoprost. The topical composition may comprise from about 0.05 % (w/v) to about 10 % (w/v) or from about 0.1 % (w/v) to about 5 % (w/v) bimatoprost. In preferred embodiments of the invention the topical composition may comprise about 3% (w/v) bimatoprost.

Suitable progesterone for use in the present invention may be natural or synthetic. However, natural progesterone is preferred. Hair thinning can be a symptom of oestrogen dominance in women or oestrogen excess in men. Oestrogens endogenous or can be introduced from environmental sources such as commercially raised meat, spermicide, detergents, soft plastics, pesticides, herbicides, personal care products and even tap water. Progesterone can be helpful to redress oestrogen imbalances, whether due to intrinsic or extrinsic sources, or even a combination of the two.

In embodiments of the invention the topical composition comprises at least about 0.5 % (w/v) progesterone or at least about 1 % (w/v) progesterone or at least about 5 % (w/v) progesterone. The topical composition may comprise from about 0.5 % (w/v) to about 15 % (w/v) progesterone or from about 1 % (w/v) to about 10 % (w/v). In preferred embodiments of the invention the topical composition may comprise about 5 % (w/v) progesterone.

The topical composition may comprise one or more additional components such as a polyphenol and/or an α-amino acid.

Polyphenols are known to have antioxidant activity and the inventor of the present therapy has recognised that this may provide benefits in reducing or preventing damage to hair follicles. Indeed, antioxidants reduce the activity of Protein Kinase C (PKC), an enzyme which has been identified as a negative regulator of hair follicle growth and which may play a part in the transduction of follicular growth-inhibitory signals. Polyphenols also reduce or prevent inflammation through their activity on the tumour necrosis factor (TNF-o) pathway. Certain polyphenols, such as those obtained from grape seed extract, are also believed to be nitric oxide releasers. Polyphenols may also be provided in the form of proanthocyanidins, which are known to act as growth stimulants for hair follicles.

Suitable polyphenols for use in the present invention may be natural or synthetic, but natural polyphenols are preferred. Preferably the polyphenols are plant-based. The polyphenol may be provided in the form of a polyphenolic extract, for example from grape skin, grape seeds, berries, olive pulp or maritime pine bark. In preferred embodiments of the invention the polyphenol is provided in the form of grape seed extract. Preferably the grape seed extract comprises at least 90 % (w/v) oligomeric proanthocyanidin complexes (OPCs) or at least 40% w/v proanthocyanidins. Suitable polyphenols additionally include resveratrol.

The topical composition may comprise at least about 0.5 % (w/v) polyphenol or at least about 1 % (w/v) polyphenol or at least about 5 % (w/v) polyphenol. The topical composition may comprise from about 0.5 % (w/v) to about 15 % (w/v) polyphenol or from about 1 % (w/v) to about 10 % (w/v). In preferred embodiments of the invention the topical composition may comprise about 5 % (w/v) polyphenol.

Certain α-amino acids, such as arginine or citrulline, are known to increase nitric oxide activity and can therefore act as vasodilators, improving blood supply to hair follicles and may contribute to hair regrowth by stimulating the production of new hair follicles.

The topical composition may comprise at least about 0.5 % (w/v) α-amino acid or at least about 1 % (w/v) α-amino acid or at least about 5 % (w/v) α-amino acid. The topical composition may comprise from about 0.5 % (w/v) to about 15 % (w/v) α-amino acid or from about 1 % (w/v) to about 10 % (w/v). In preferred embodiments of the invention the topical composition may comprise about 5 % (w/v) α-amino acid.

The α-amino acid may be one or more of arginine or citrulline, preferably L-arginine or L-citrulline or a combination thereof.

The topical composition may additionally comprise an essential oil and/or a 5o-reductase inhibitor.

The essential oil may be rosemary oil. When applied topically rosemary can promote new cell growth on the scalp, at least in part by curbing the build-up of sebum, which can plug hair follicles. Rosemary also contains an antioxidant and anti-inflammatory compound known as rosmarinic acid, which is believed to reduce age-related damage to the body's protein structures.

5α-reductase inhibitors are known to have anti-androgenic activity. These compounds inhibit the enzyme 5o-reductase, which is most known for conversion of testosterone to DHT. 5o-reductase inhibitors, such as finasteride, have been successfully used in topical compositions for treating hair loss. Suitable 5α-reductase inhibitors may be plant-based and may comprise one or more of Reishi mushroom, saw palmetto, green tea or extracts or combinations thereof. Both progesterone and grape seed extract are known to have 5a-reductase inhibitory activity in addition to the other specific activities mentioned above and can therefore be used as 5a-reductase inhibitors. Preferably the 5a-reductase inhibitor is not finasteride.

The topical composition may therefore comprise bimatoprost, progesterone, a polyphenol, such as grape seed extract, an alpha amino acid, such as L-arginine and an essential oil, such as rosemary essential oil. The topical composition may additionally comprise a 5a-reductase inhibitor, such as saw palmetto.

The topical composition may additionally comprise magnesium. Suitable forms of magnesium will be familiar to the skilled person and may include magnesium oxide, magnesium chloride, magnesium chloride hexahydrate, magnesium disodium ethylenediaminetetraacetate (EDTA) and/or magnesium citrate. Magnesium interacts with calcium in many body processes, such as regulation of blood vessels, contraction of muscles. Calcium stimulates contraction of muscles and blood vessels, while magnesium relaxes muscles and dilates blood vessels. Magnesium therefore has a vasodilatory activity. Magnesium may also have a calcium chelating effect in the scalp, which may provide beneficial effects in terms of treating hair loss or stimulating hair regrowth.

The topical composition may additionally comprise Vitamin K2 (also known as menaquinone). Similarly to magnesium, Vitamin K2 has calcium chelating activity, which may provide beneficial effects in terms of treating hair loss or stimulating hair regrowth. Vitamin K2 has several subtypes, which are generally divided into the short-chain menaquinones (such as MK-4) and the long-chain menaquinones, including MK-7, MK-8 and MK-9. MK-7 is particularly preferred in compositions described herein.

The enteric composition comprises a vasodilator. As explained above, vasodilators can be beneficial in treating hair loss by improving blood supply to hair follicles, and may contribute to hair regrowth by stimulating the production of new hair follicles.

The vasodilator comprises minoxidil. Minoxidil is a prodrug that is converted by sulfation via the sulfotransferase enzyme SULT1A1 to its active form, minoxidil sulfate.

Minoxidil is a potassium channel opener, causing hyperpolarization of cell membranes. Hypothetically, by widening blood vessels and opening potassium channels, it allows more oxygen, blood, and nutrients to the hair follicles. This may cause follicles in the telogen phase to shed, which are then replaced by thicker hairs in a new anagen phase. Minoxidil contains the nitric oxide chemical moiety and acts as a nitric oxide agonist, which is known to have vasodilatory activity.

Other vasodilators include α-amino acids, such as arginine or citrulline, preferably L-arginine or L-citrulline. α-amino acids may be included in addition to the vasodilators described above.

The enteric composition preferably comprises at least about 0.5 mg of minoxidil or at least about 1 mg of minoxidil or at least about 5 mg of minoxidil. The topical composition may comprise from about 0.5 mg to about 15 mg of minoxidil or from about 1 mg to about 10 mg of minoxidil. In preferred embodiments of the invention the topical composition may comprise about 5 mg or about 2 mg of minoxidil.

The enteric composition additionally comprises zinc, grape seed extract and L-citrulline.

Zinc can act as a 5o-reductase inhibitor, reducing or preventing the conversion of testosterone to DHT. Zinc has also been shown to be an aromatase inhibitor, which can reduce oestrogen production within the body. Zinc also has antioxidant activity. All of these can be beneficial in the therapy of the present invention for at least the reasons discussed herein.

The enteric composition may comprise at least about 1 mg zinc, or at least about 10 mg zinc or at least about 30 mg zinc. The enteric composition may comprise from about 1 mg to about 100 mg of zinc or from about 10 mg to about 60 mg of zinc. In preferred embodiments of the invention the enteric composition may comprise about 30 mg of zinc.

The enteric composition may comprise at least about 50 mg L-citrulline or at least about 100 mg L-citrulline or at least about 500 mg L-citrulline. The enteric composition may comprise from about 50 mg to about 1000 mg L-citrulline or from about 100 mg to about 1000 mg. In preferred embodiments of the invention the topical composition may comprise about 500 mg L-citrulline acid.

The enteric composition may additionally comprise magnesium. Suitable forms of magnesium will be familiar to the skilled person and may include magnesium oxide, magnesium chloride, magnesium chloride hexahydrate, magnesium disodium ethylenediaminetetraacetate (EDTA) and/or magnesium citrate. Magnesium interacts with calcium in many body processes, such as regulation of blood vessels, contraction of muscles. Calcium stimulates contraction of muscles and blood vessels, while magnesium relaxes muscles and dilates blood vessels. Magnesium therefore has a vasodilatory activity. Magnesium may also have a calcium chelating effect in the scalp, which may provide beneficial effects in terms of treating hair loss or stimulating hair regrowth.

The enteric composition may additionally comprise Vitamin K2 (also known as menaquinone). Similarly to magnesium, Vitamin K2 has calcium chelating activity, which may provide beneficial effects in terms of treating hair loss or stimulating hair regrowth. Vitamin K2 has several subtypes, which are generally divided into the short-chain menaquinones (such as MK-4) and the long-chain menaquinones, including MK-7, MK-8 and MK-9. MK-7 is particularly preferred in compositions described herein.

The enteric composition may comprise at least about 50 mg grape seed extract or at least about 100 mg grape seed extract or at least about 250 mg grape seed extract. The enteric composition may comprise from about 50 mg to about 500 mg grape seed extract I or from about 100 mg to about 500 mg. In preferred embodiments of the invention the topical composition may comprise about 250 mg grape seed extract.

Preferably the grape seed extract comprises at least 90 % (w/v) oligomeric proanthocyanidin complexes (OPCs) or at least 40% (w/v) proanthocyanidins.

The enteric composition may additionally comprise a 5o-reductase inhibitor. 5α-reductase inhibitors are preferably plant-based and may comprise one or more of Reishi mushroom, saw palmetto, green tea or extracts or combinations thereof. Preferably the 5o-reductase inhibitor is not finasteride.

The enteric composition therefore comprises minoxidil, an L-citrulline, grape seed extract, and zinc. The enteric composition may additionally comprise a 5o-reductase inhibitor, such as saw palmetto.

The topical and enteric compositions are preferably administered sequentially. The topical composition may be administered before the enteric composition. Alternatively, the enteric composition may be administered before the topical composition.

The interval between administering the topical and enteric compositions is preferably not more than 12 hours, or 24 hours, or 48 hours. Preferably the interval between administering each composition is 24 hours or less, or 12 hours or less. The topical and enteric compositions may be administered at least once every three days, or at least once every two days. Preferably the topical and enteric compositions are to be administered at least once daily. The topical composition may be administered once or twice per day. The enteric composition may be administered once per day or once every two days. Optionally, the enteric composition may be administered twice per day. The combination therapy may be administered indefinitely in order to provide continued support of existing hair follicles and the maintenance of any experienced hair regrowth.

Topical and enteric compositions of the present invention may additionally include one or more pharmaceutically acceptable carriers and/or excipients, such as diluents, adjuvants, vehicles, fillers, binders, disintegrating agents, wetting agents, emulsifying agents, suspending agents, perfuming agents, buffers, dispersants, thickeners, solubilising agents, lubricating agents and dispersing agents, depending on the nature of the mode of administration and dosage forms. In embodiments of the invention the compositions may be sterile.

The topical composition may be provided in the form of a lotion, a gel, an ointment, a cream, a foam, a transdermal patch, a dressing, a powder, a paste or a tinctures. In preferred embodiments of the invention the topical composition may be in the form of a lotion.

The enteric composition may take the form, for example, of a solid preparation including tablets, capsules, drageés, lozenges, granules, powders, pellets and cachets; and liquid preparations including gels, lotions, suspensions, elixirs, syrups, suspensions, sprays, emulsions and solutions. In preferred embodiments of the invention the enteric composition is in the form of a capsule.

The hair loss may be caused by alopecia, especially androgenic alopecia. The hair loss may be caused by male-pattern hair loss, female-pattern hair loss, alopecia areata, traction alopecia or telogen effluvium.

The subject is a mammal, preferably a human, and may be male or female. The subject may be an adult human. Preferably the subject is an adult human male.

### Brief Description of the Drawings

The invention will now be described in detail, by way of example only, with reference to the figures.
Figure 1 shows the effects of the combination therapy of the present invention after 10 weeks of once daily administration in a 62 year old man (Figure 1A shows hair density before treatment and Figure 1B shows hair density after treatment).
Figure 2 shows the effects of the combination therapy of the present invention after 8 weeks of once daily administration in a 26 year old man (Figure 2A shows the scalp before treatment and Figure 2B shows the scalp after treatment).
Figure 3 shows the effects of the combination therapy of the present invention after 8 weeks of once daily administration in a 50 year old man (Figures 3A and B show the front of the scalp before and after treatment; Figures 3C and D show the back of the scalp before and after treatment).
Figure 4 shows the effects of the combination therapy of the present invention after 3 months of once daily administration in a male subject (Figure 4A shows the scalp before treatment and Figure 4B shows the scalp after treatment).
Figure 5 shows the effects of the combination therapy of the present invention after 6 months of once daily administration in a male subject (Figure 5A shows the scalp before treatment and Figure 5B shows the scalp after treatment).
Figure 6 shows the effects of the combination therapy of the present invention after 8 weeks of once daily administration of enteric preparation and twice daily application of the lotion in a male subject (Figure 6A shows the scalp before treatment and Figure 6B shows the scalp after treatment).

### Example 1

A combination therapy comprising enteric and topical compositions comprising the BioGroHair^{®} treatment was administered to volunteers. The enteric and topical compositions were provided as a prescription supplement (in the form of a capsule) and a lotion, both formulated as outlined below. Both were taken once a day. The principle behind the BioGroHair^{®} treatment involves improving blood flow to the scalp, reducing oxidative stress and inflammation on the hair follicles and preventing the hair shedding effect of excessive Dihydrotestosterone.

Lotion:
1. Grape Seed Extract (GSE) 5% (w/v)
2. Rosemary -0.1% (w/v) (as essential oil)
3. L-arginine- 5% (w/v)
4. Bimatoprost 0.3% (w/v) (0.3mg/ml)
5. Progesterone 0.5% w/v (5mg/ml)

Supplement (capsule):
1. GSE 250mg (labelled to contain greater than 90% OPCs and at least 40% proanthocyanidins).
2. Minoxidil 5mg
3. L-citrulline 500mg
4. Zinc 30mg

### Results

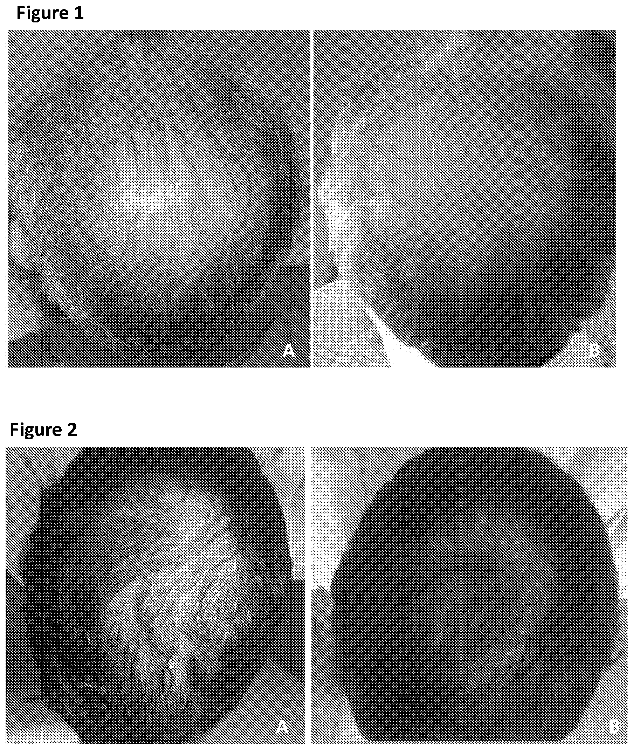

Figure 1 show the effects of the BioGroHair^{®} treatment after only 10 weeks in a 62 year old man (Figure 1A shows hair density before treatment and Figure 1B shows hair density after treatment).

The user testified that:
"I used the BioGro products for 2 months as part of the Natural Doctor clinic trial. I was very pleased with the results. My hair was clearly thicker and visibly much fuller. I must admit I was so sceptical at first but I was so pleasantly surprised by the results"
XD, 62 Y.O.

Figure 2 shows the effects of the BioGroHair^{®} treatment after only 8 weeks in a 26 year old man (Figure 2A shows the scalp before treatment and Figure 2B shows the scalp after treatment).

The user testified that:
"Because of my height and my fun personality I don't think anyone noticed that I was self-conscious about the thinning of my hair; however I wasn't willing to try surgery to fix it. I heard about the BioGroHair^{®} program from a friend and, if I'm being honest I was quite sceptical when I heard about it.
I was sceptical because I'd had to deal with my hair thinning from such a young age that I didn't think lotion and supplements would make a difference. But I was pleasantly surprised at my results and the program was so easy to follow!
For the first three months I took two tablets - usually in the morning with my breakfast before work - and rubbed the lotion on my scalp every night before I went to bed. It dried really quickly and didn't leave any stains or marks on my pillows.
I had a holiday booked around the 2 and a half month mark, and whilst I was packing up my lotion and supplements I decided to have a closer peer than usual at my hair, and was genuinely shocked at how much of a difference the program had made after such a short amount of time. It's now been 4 months since I've been on the program and I've started to reduce how often I use the lotion and supplements to around 2 times a week. My friends and family are always commenting on how thick and full my hair looks now, saying I also look younger and happier.
I would definitely recommend the BioGroHair^{®} program because it gets to work really quickly and I was pleasantly surprised with my results after just putting up with my thinning hair for 10 years."
User 2, 26 Y.O.

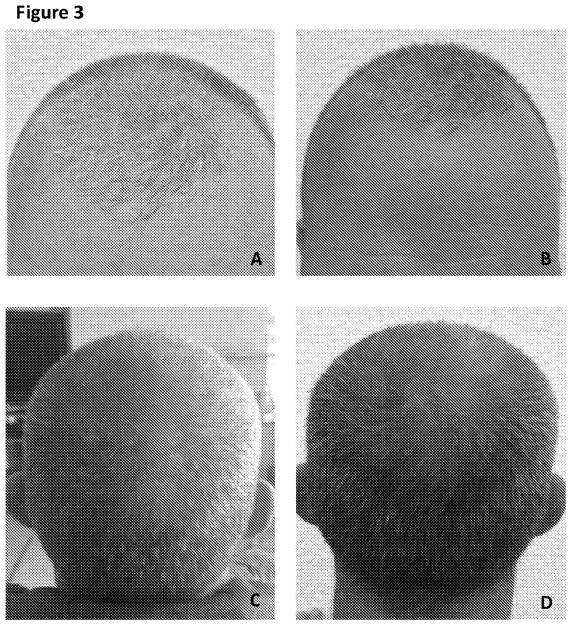

Figure 3 shows the effects of BioGroHair^{®} treatment after only 8 weeks in a 50 year old man (Figures 3A and B show the front of the scalp before and after treatment; Figures 3C and D show the back of the scalp before and after treatment).

The user testified:
"A quick update to the treatment capsules and ointment for hair loss I am currently using. Although it has only been 2 weeks, both myself and my wife have noticed, although only minor, the hair growth has certainly become thicker, more of a covering. This is very encouraging, following many years of not really being bothered about my hair loss, now that I am noticing an improvement I am very excited at the prospects of once again having a full head of hair.
I will continue to update you over the next 10 weeks and thanks again for giving me the opportunity to use your excellent product."
AP, 50 Y.O.

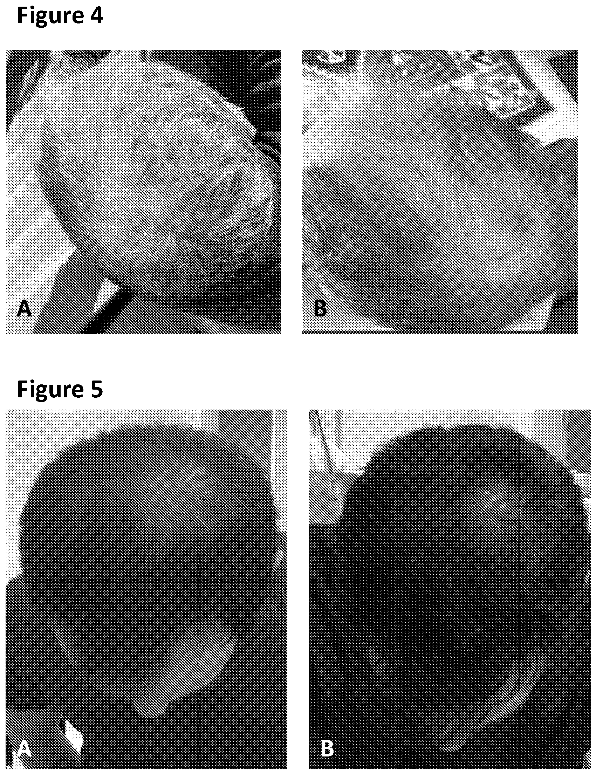

Figure 4 shows the effects of the combination therapy of the present invention after 3 months of once daily administration in a male subject (Figure 4A shows the scalp before treatment and Figure 4B shows the scalp after treatment).

The user testified:
"In my personal opinion the products are easy to use... By 3 months it seems my hair was definitely thicker on top, which encouraged me to continue with another three months worth of products. I would recommend it to others to try as it is nice to feel I am able to do something to save the hair I have and increase it a little."

Figure 5 shows the effects of the combination therapy of the present invention after 6 months of once daily administration in a male subject (Figure 5A shows the scalp before treatment and Figure 5B shows the scalp after treatment).

The user testified:
"It has worked fantastically; the change has been incredible".

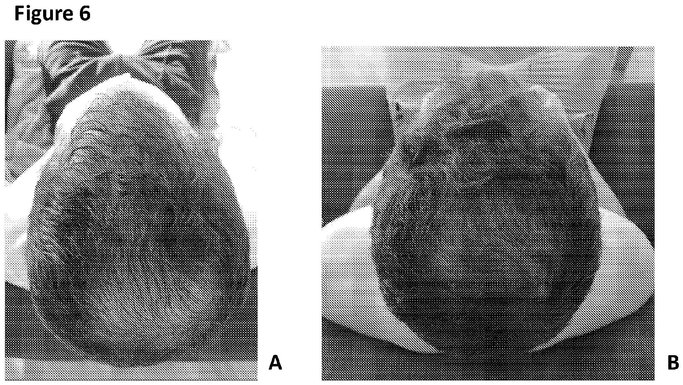

Figure 6 shows the effects of the combination therapy of the present invention after 8 weeks of once daily administration of enteric preparation and twice daily application of the lotion in a male subject (Figure 6A shows the scalp before treatment and Figure 6B shows the scalp after treatment).

The user testified that:
"The product is very easy to use and I've built it into my morning and evening routine. Applying it on the scalp takes a few seconds each time and the supplement is easy to consume as well. I had no major expectations and am positively surprised by how much my hair really has grown back. I started noticing a few new hairs grow back after one month. After two months the before and after pictures started to show a significant improvement as the back of my head is much more covered now. My hairline also looks fuller and I spend more time styling my hair than worrying about where it all went! Love the fact that it's all natural and non-invasive. Worth a try and I highly recommend it."
RS, 33 Y.O.

## Claims

1. A combination therapy comprising:
(a) a topical composition comprising bimatoprost and progesterone; and
(b) an enteric composition comprising minoxidil, L-citrulline, grape seed extract and zinc,
for use in treating hair loss.

2. The combination therapy for use according to claim 1, wherein the topical composition comprises at least about 0.05 % (w/v) bimatoprost and/or at least about 1 % (w/v) progesterone.

3. The combination therapy for use according to claim 1, wherein the topical composition additionally comprises at least about 1 % (w/v) grape seed extract.

4. The combination therapy for use according to any of claims 1 to 3, wherein the topical composition additionally further comprises an essential oil and/or a 5o-reductase inhibitor.

5. The combination therapy for use according to any of claims 1 to 4, wherein the enteric composition comprises at least about 1 mg minoxidil.

6. The combination therapy for use according to claim 1, wherein the enteric composition comprises at least about 1 mg zinc, and/or at least about 100 mg grape seed extract and/or at least about 100 mg L-citrulline.

7. The combination therapy for use according to any of claims 1 to 6, wherein the enteric composition additionally comprises a 5o-reductase inhibitor.

8. The combination therapy for use according to any of claims 1 to 7, wherein the topical and systemic compositions are to be administered at least once daily.

9. The combination therapy for use according to any of claims 1 to 8, wherein the topical composition is in the form of a lotion, and/or wherein the enteric composition is in the form of a capsule.

10. The combination therapy for use according to any of claims 1 to 9, wherein the hair loss is caused by alopecia, especially androgenic alopecia.

11. A cosmetic non-therapeutic method for reducing hair loss or stimulating hair growth or regrowth, the method comprising administering a combination therapy comprising:
(a) a topical composition comprising bimatoprost and progesterone; and
(b) an enteric composition comprising minoxidil, L-citrulline, grape seed extract and zinc,
to a subject in need thereof.

## Patentansprüche

1. Kombinationstherapie, die Folgendes umfasst:
(a) eine topische Zusammensetzung, die Bimatoprost und Progesteron enthält; und
(b) eine enterale Zusammensetzung, die Minoxidil, L-Citrullin, Traubenkernextrakt und Zink enthält,
zur Verwendung bei der Behandlung von Haarausfall.

2. Kombinationstherapie zur Verwendung nach Anspruch 1, wobei die topische Zusammensetzung mindestens etwa 0,05 % (w/v) Bimatoprost und/oder mindestens etwa 1 % (w/v) Progesteron enthält.

3. Kombinationstherapie zur Verwendung nach Anspruch 1, wobei die topische Zusammensetzung zusätzlich mindestens etwa 1 % (w/v) Traubenkernextrakt enthält.

4. Kombinationstherapie zur Verwendung nach einem der Ansprüche 1 bis 3, wobei die topische Zusammensetzung ferner zusätzlich ein ätherisches Öl und/oder einen 5α-Reduktase-Inhibitor enthält.

5. Kombinationstherapie zur Verwendung nach einem der Ansprüche 1 bis 4, wobei die enterale Zusammensetzung mindestens etwa 1 mg Minoxidil enthält.

6. Kombinationstherapie zur Verwendung nach Anspruch 1, wobei die enterale Zusammensetzung mindestens etwa 1 mg Zink und/oder mindestens etwa 100 mg Traubenkernextrakt und/oder mindestens etwa 100 mg L-Citrullin enthält.

7. Kombinationstherapie zur Verwendung nach einem der Ansprüche 1 bis 6, wobei die enterale Zusammensetzung zusätzlich einen So-Reduktase-Inhibitor enthält.

8. Kombinationstherapie zur Verwendung nach einem der Ansprüche 1 bis 7, wobei die topischen und systemischen Zusammensetzungen mindestens einmal täglich verabreicht werden sollen.

9. Kombinationstherapie zur Anwendung nach einem der Ansprüche 1 bis 8, wobei die topische Zusammensetzung in Form einer Lotion vorliegt und/oder die enterale Zusammensetzung in Form einer Kapsel vorliegt.

10. Kombinationstherapie zur Anwendung nach einem der Ansprüche 1 bis 9, wobei der Haarausfall durch Alopezie, insbesondere androgene Alopezie, verursacht wird.

11. Kosmetisches, nicht-therapeutisches Verfahren zur Verringerung des Haarausfalls oder zur Stimulierung des Haarwachstums oder des Nachwachsens,
wobei das Verfahren die Verabreichung einer Kombinationstherapie umfasst, die Folgendes umfasst:
(a) eine topische Zusammensetzung, die Bimatoprost und Progesteron umfasst, und
(b) eine enterale Zusammensetzung, die Minoxidil, L-Citrullin, Traubenkernextrakt und Zink enthält,
an ein Subjekt, das dessen bedarf.

## Revendications

1. Une thérapie combinée comprenant :
(a) une composition topique comprenant du bimatoprost et de la progestérone ; et
(b) une composition entérique comprenant du minoxidil, de la L-citrulline, de l'extrait de pépins de raisin et du zinc, destinée à être utilisée dans le traitement de la chute des cheveux.

2. La thérapie combinée à utiliser selon la revendication 1, dans laquelle la composition topique comprend au moins environ 0,05 % (p/v) de bimatoprost et/ou au moins environ 1 % (p/v) de progestérone.

3. La thérapie combinée à utiliser selon la revendication 1, dans laquelle la composition topique comprend en outre au moins environ 1 % (p/v) d'extrait de pépins de raisin.

4. La thérapie combinée à utiliser selon l'une quelconque des revendications 1 à 3, dans laquelle la composition topique comprend en outre une huile essentielle et/ou un inhibiteur de la 5α-réductase.

5. La thérapie combinée à utiliser selon l'une quelconque des revendications 1 à 4, dans laquelle la composition entérique comprend au moins environ 1 mg de minoxidil.

6. La thérapie combinée à utiliser selon la revendication 1, dans laquelle la composition entérique comprend au moins environ 1 mg de zinc et/ou au moins environ 100 mg d'extrait de pépins de raisin et/ou au moins environ 100 mg de L-citrulline.

7. La thérapie combinée à utiliser selon l'une quelconque des revendications 1 à 6, dans laquelle la composition entérique comprend en outre un inhibiteur de la 5α-réductase.

8. La thérapie combinée à utiliser selon l'une quelconque des revendications 1 à 7, dans laquelle les compositions topiques et systémiques doivent être administrées au moins une fois par jour.

9. La thérapie combinée à utiliser selon l'une quelconque des revendications 1 à 8, dans laquelle la composition topique est sous la forme d'une lotion, et/ou dans laquelle la composition entérique est sous la forme d'une capsule.

10. La thérapie combinée à utiliser selon l'une quelconque des revendications 1 à 9, dans laquelle la perte de cheveux est provoquée par une alopécie, en particulier une alopécie androgénique.

11. Méthode cosmétique non thérapeutique pour réduire la chute des cheveux ou stimuler la croissance ou la repousse des cheveux, la méthode comprenant l'administration d'une thérapie combinée comprenant :
(a) une composition topique comprenant du bimatoprost et de la progestérone ; et
(b) une composition entérique comprenant du minoxidil, de la L-citrulline, de l'extrait de pépins de raisin et du zinc,
à un sujet en ayant besoin.
